# EUROPEAN PATENT SPECIFICATION

(11) **EP 2 155 831 B1**
(45) Date of publication and mention of the grant of the patent: **26.09.2012**
(21) Application number: 08760528.3
(22) Date of filing: 04.06.2008
(51) Int. Cl.: C09J 7/02, A61F 13/02

(54) **A METHOD FOR MANUFACTURING TWO DIFFERENT PATTERNED ADHESIVE LAYERS SIMULTANEOUSLY**
VERFAHREN ZUR GLEICHZEITIGEN HERSTELLUNG ZWEIER VERSCHIEDENER STRUKTURIERTER HAFTSCHICHTEN
PROCÉDÉ DE FABRICATION SIMULTANÉE DE DEUX COUCHES ADHÉSIVES À MOTIFS DIFFÉRENTS

(30) Priority: 04.06.2007 DK 200700810
(43) Date of publication of application: 24.02.2010
(73) Proprietor: Coloplast A/S, 3050 Humlebæk (DK)
(72) Inventor: BOUGHERARA, Chaabane, DK-2000 Frederiksberg C (DK); LAM, Peter Kwok Hing, DK-2000 Frederiksberg C (DK); STRØM-HANSEN, Bjarke, DK-2750 Ballerup (DK)
(86) International application number: PCT/EP2008/056949
(87) International publication number: WO 2008/148827

(56) References cited:
- EP-A- 0 918 080
- DE-A1- 2 900 319

## Description

### FIELD OF THE INVENTION

This invention relates to medical adhesive tapes for use in adhering medical appliances, dressings, etc., to the skin. More specifically, the present invention is directed to a method of manufacturing a medical adhesive tape and dressing, having high vapour permeability and liquid resistance at the same time.

### BACKGROUND OF THE INVENTION

In medical fields, medical adhesive tapes such as surgical tapes, plasters (first-aid plasters), etc., are applied to adhere medical appliances, rolled bandages, wound dressings, transdermal absorbents, etc., to the skin.

The adhesive tape is composed of an adhesive which is coated on a backing layer. Such an adhesive tape should have adhesion suitable for firmly adhering the medical appliances or dressing materials to the skin and subsequently easily removing them from the skin. Furthermore, the tape should have high water vapour permeability to avoid normal skin damage because it is directly attached to the skin, in which high water vapour permeability can promote wound healing.

Conventionally, the adhesive tape has the pressure-sensitive adhesive (PSA) coated onto an entire surface of the backing layer. Thus, conventional tapes are disadvantageous in terms of low water vapour permeability, due to the coated adhesive per se, regardless of the water vapour permeability of the backing layer. That is, even though a backing layer having high water vapour permeability is used, the adhesive coated on the entire surface of the backing layer may prevent water vapour permeation, whereby the water vapour permeability of the tape is reduced.

The user of the adhesive tape may come into contact with water. Hence, water or liquid resistance is required to protect the wound or medical appliance.

Dot-shaped patterns of pressure sensitive adhesives (PSA) coated on backing film allow increased vapour permeability at the areas which are not coated. However, water infiltration or leakage is possible via the interconnected uncoated areas or channels, which are defined between the non-interconnected dots of a dot-shaped adhesive pattern.

Specifically, dots of the dot-shaped adhesive pattern are discontinuously formed, that is, the dots are not interconnected. As a consequence, such designs allow water to be trapped in the space defined between the non-interconnected dots. Such trapped water may be in contact with the skin of the user over longer periods, which leads to maceration of the skin. This is harmful to the wound. US 6171648 discloses a backing material with a partial self-adhesive coating.

WO 2005/028581 discloses a medical adhesive tape, having high water vapour permeability and water resistance, characterized in that a pressure-sensitive adhesive is coated on a base sheet to form a net-shaped structure. As such, the net-shaped structure includes a continuous rectilinear form having square pores, a continuous curvilinear form having slanted square pores, a continuous form having circular pores, or combinations thereof. The adhesive tape includes surgical tape and plasters serving to adhere medical appliances, rolled bandages, wound dressings, transdermal absorbents, etc., to the skin, and can permit the passage of a gas through a plurality of non-coating parts to have high water vapour permeability, and simultaneously have water resistance and sufficient adhesion through a continuous net type coating part.

A perforated adhesive layer increases permeability, especially where the exudate is viscous and in large quantities, the holes should be made as big as feasible. The holes or perforations make them unsuitable as the top backing layer in a dressing construction. Therefore, perforated adhesives require, a multi layer construction.

A dressing may contain a central part comprising an absorbent core.

An exposed absorbent core on a backing film requires good anchorage to the backing film, especially when wet and heavy due to being soaked with exudate. By good anchorage is meant that absorption of moisture does not cause the absorbent core and the backing film to delaminate.

An exposed absorbent core surface facing the wound side maximises the exudate absorption when in place. However, longer term placement (such as for a period longer than 3-5 days) on the wound may lead to growth of tissue of the healing wound onto the core surface or more difficult removal, which in turn irritates the wound.

To overcome the removal problem, contact layers of less adhering nature are known. These contact layers are often thin films or gel layers having perforations to expose the absorbent core.

EP 633758 discloses an absorbent wound dressing having a layer of hydrophobic silicone gel which is intended to lie against the wound surface when the dressing is worn. A layer of carrier material carries the gel layer and affords the requisite strength thereto. An absorbent body is placed on that side of the carrier material and gel layer, which lie distal from the wound surface in use. The carrier material and the gel layer have mutually coinciding penetrating perforations at least within the region of the absorbent body. A fluid barrier layer is provided on that side of the dressing which lies distal from the wound surface in use.

Another function of the contact layer may be to support and hold the absorbent core in position.

These contact layers with perforations require multi step process to manufacture, perforation of film, coating, blow holes from adhesives corresponding to the perforations of the films, or make holes in the adhesive and film at the same time. Others use only the bare adhesive layer, requiring perforation and transferring. Such adhesive layer needs to be at least partially gelled for the perforations to stay but not so advanced in the gelation that it becomes difficult to adhere or laminate onto the substrate.

As mentioned above pattern coatings of adhesives or gels on backing or transfer films are well known. In any one method of production, if a change of pattern is required, it is necessary to change the tools or program for screen printing, gravure coating etc.

EP 5 532 275 discloses a non re-enforced and non-adherent dressing hydrophilic gel. The gel is manufactured by applying an aqueous solution to a mould defining a pattern of interconnected grooves and subsequently drying the solution in the mould to form the dressing.

DE 29 00 319 discloses a punching apparatus for making coiled bands with adhesives.

EP 0 437 916 discloses a method for producing an air-permeable adhesive tape by forming a layer of a solution on a substrate, the solution comprising an adhesive in an organic solution; applying water drops on said layer and evaporation the organic solvent contained so as to form an adhesive that contain water drops and finally evaporating the water. However, it will be appreciated, that the size and distribution of the water droplets are difficult to control.

As use of organic solvents is associated with environmental and health problems, it is an object of a preferred embodiment, to provide a method in which the adhesive does not contain organic solvents.

US 2005/0228329 discloses polymer material which in order to be gelled must be cooled down from a heated state. It will be appreciated that such heating and subsequently cooling is time consuming and accordingly, it is an object of a preferred embodiment of the present invention to provide a less time consuming method/process.

There is a need to simply the processes of making patterned and perforated adhesive layers. There is also a need to be able to use the advantages of using the different types of coatings, dot, net, or perforated, without having their short comings.

### SUMMARY OF THE INVENTION

The present invention relates to a method of manufacturing two different patterned adhesive layers simultaneously, and a wound dressing comprising at least one of the adhesive layers.

### BRIEF DESCRIPTION OF THE FIGURES

Figure 1 illustrates a continuous manufacturing method of the invention,
Figure 2 illustrates a top view of a top coated layer manufactured by the method illustrated in Figure 1,
Figure 3 illustrates a top view of a bottom coated layer manufactured by the method illustrated in Figure 1,
Figure 4 is a top view showing an embodiment of the invention where a construction of a dressing utilising the advantages to the two types of adhesive sheets formed by the method illustrated in Figure 1,
Figure 5 illustrates a sectional view taken along a line in the middle of the dressing illustrated in Figure 4,
Figure 6 illustrates a sectional view of another embodiment of the invention,
Figure 7 illustrates a sectional view of an embodiment of the invention, and
Figure 8 illustrates a sectional view of yet another embodiment of the invention.

### DETAILED DESCRIPTION OF THE INVENTION

In a FIRST aspect, the present invention relates to a method for manufacturing a first and a second backing layer each of which carrying on an upper surface an adhesive layer defining a predetermined pattern, the first backing layer defining one or more indentations through each of which an adhesive coated onto the upper surface of the first backing layer may flow onto a predetermined area of the upper surface of the second layer when the first backing layer is provided on the upper surface of the second backing layer, the method comprising the steps of:
- providing the first backing layer on the upper surface of the second backing layer;
- coating the adhesive onto the upper surface of the first backing layer and the predetermined areas of the second backing layers;
- separating the first and second backing layers so as to define the predetermined pattern on the upper surface of each of the first and second backing layer.

The current invention provides a one step process of making two adhesive layers simultaneously. A first adhesive layer provided in an upper surface of the first backing layer and a second adhesive layer provided on an upper surface of the second backing layer. It will be appreciated that the first and second adhesive layers complement each other such that an adhesive zone is defined in the second adhesive layer where a protrusion is defined at the corresponding position of the first adhesive layer. By corresponding position is meant that during manufacture, the area of the perforation (and thus also the indentation in the first backing layer) overlapped the adhesive zone (and thus also the predetermined zone in the second backing layer).

Depending on the geometry of the indentations in the first (the upper) backing layer, the first adhesive layer will define a plurality of perforations each of which defines a rim portion which is constituted by the adhesive layer. Additionally depending on the geometry of the indentations in the first (the upper) backing layer, the second adhesive layer will define a plurality of discrete adhesive zones which are not interconnected. It will be appreciated that the pattern of the first adhesive layer is the inverse pattern of the second adhesive layer, and vice versa.

According to an embodiment, the first (upper) layer of adhesive is a connected layer with perforations, i.e. a sheet of adhesive defining one or more perforations.

In one embodiment, the step of coating the adhesive comprises the step of retaining the first backing layer on the upper surface of the second backing layer. Such retaining may be achieved by one or more of the following methods/means. The first and second layers may be glued together with an adhesive, which is sufficiently strong to keep the two layers together, while allowing them to be separated easily, e.g. by having a peel force which is lower than the separation force required to separate the first and second adhesive layers from each other.

In another embodiment, the second (lower) backing layer comprises a plurality of perforations positioned below areas of the first (upper) backing layer which allows the upper layer to be sucked onto the second layer when a vacuum is provided below the second (lower) layer. Moreover, the two layers may be held together by electrostatic forces. In one embodiment, a layer of liquid film (possibly as thin as possible) is provided between the two layers, whereby the two layers are held together due to the surface tension between the liquid and each of the two backing layers. Moreover, rollers may be utilised to force the two layers together.

In one embodiment, the step of separating causes:
- the first adhesive layer to be defined on the upper surface of the first backing layer, the first adhesive layer defining a perforation in the area of each of the indentations in the first backing layer; and
- the second adhesive layer to be defined on the upper surface of the second backing layer, the second adhesive layer defining discrete adhesive zones in each of the predetermined areas of the second backing layer.

In one embodiment, one or both of the backing films may be perforated. When both films are perforated, the films are arranged such that the holes/perforations do not coincide during the adhesive coating process. By providing perforations in the second backing layer, the permeability of the final product is increased as vapour may permeate through the perforation in the second backing layer.

The shape of the discrete dots of the second layer coincides with the shape of the perforations of the first layer.

The adhesive layer may be a so called transfer coating in which the adhesive is provided between two release liners such that the coating is suitable for being transferred onto a surface of the final product while leaving one of the surfaces coated with one of the release liners.

In another embodiment, the backing film is a permanent film suitable as backing material for the adhesive in a dressing. By permanent film is meant a film which is used in a final product e.g. a wound dressing this is contrary to a release liner of a transfer film/coating which is adapted to be removed prior to application of the adhesive to a surface of the final product. It will be appreciated that in the case of a permanent backing film which is provided on one surface of the adhesive, a release liner may be provided on the opposite surface of the adhesive. The release liner may be removed prior to application of the final product (including the permanent backing film and the adhesive).

The backing film may be pre-treated, chemically or by corona treatment etc, to improve anchoring properties. Examples are silicone and titanate adhesion promoters.

The first and second backing layers may comprise identical materials/components. In one embodiment the two backing layers are identical. In another embodiment the first and second backing layers do not comprise identical materials/components. Thus, the backing materials may be different from one another.

The layers of the adhesives may have the same or different thickness depending on the speed of operation, and ability to flow. The thickness of the first adhesive is typically 50% - 100% of that of the second.

In one embodiment, the thickness of the adhesive layer is between 25 to 2000µm, preferably 50 to 1000µm.

The adhesive is in liquid form during coating. In one embodiment, the adhesive layer is additionally in liquid form during separation of the two coated backing layers, and becomes a gel (form-stable) immediately (typically within less than a few minutes) afterwards, by cooling, heating, reacting or crosslinking. In another embodiment, the adhesive layer has reached a form-stable state in the area of separation upon separation of the two backing layers.

Each of the perforations in the first adhesive layer may define a rim portion. In one embodiment, the rim portion is defined while the rim portion is in liquid form. Thus the method may, subsequent to the step of separating, comprise the step of curing the first and second adhesive layer. In another embodiment the rim portion is defined when the rim portion is in a form-stable form. Thus the method may, prior to the step of separating, comprise the step of curing the first adhesive layer at least in the area of the rim portion.

The adhesive is in a liquid form during coating and becomes a form-stable mass upon coating. The adhesive may be a two-component system. Preferably, the adhesive contains no solvent. Preferred adhesives include PU, acrylic, silicone (e.g. Silbione RTgel 4512 (Rhodia), Dow Corning 7-9800), or polyethylene or polypropylene oxide based crosslinking types as described in patent WO2005/032401. The adhesive may be a hotmelt type, which is initially heated to flow and cooled to gel or crosslink. Instead of curing upon cooling the adhesive may in some embodiments cure upon application of thermal energy or energy from another energy source.

In one embodiment, the method (prior to the step of coating) comprises the step of priming at least a part of the upper surface of the first and/or second backing layer with a primer adapted to enhance the binding/anchorage between the adhesive and the respective surface(s). In one embodiment, the primer is chosen such that the force needed to separate the two adhesive layers in the area of the rim portion of the perforations of the first upper layer and the adhesive zones of the second lower layer, is lower than the force (the peel force) needed to separate the adhesive from the backing layer to which it is attached.

It will be appreciated that while separation of the two layers causes the first (upper) adhesive layer to be forced downwards and towards its backing layer, the opposite is the case for the second (lower) adhesive layer. In the case of the second (lower) adhesive layer, the layer (i.e. each of the discrete adhesive zones) is forced upwards i.e. away from the second (lower) backing layer. This creates a risk of the second adhesive layer delaminating from the second backing layer. However, application of a primer may reduce or even eliminate this problem as such a primer increases the binding/anchorage between the second adhesive layer and the second backing layer.

After curing and separation, the two adhesives (i.e. the upper and lower layer of adhesive) may have different peel forces, depending on thickness and curing profile. By curing profile is meant the time and temperature necessary for completely curing the adhesive. It will be appreciated that the thicker the layer of an adhesive is, the higher is the peel force

The peel force of one or both of the adhesives may be below 10 N/cm, such as below 8 N/cm, such as below 6 N/cm, such as below 2 N/cm. It is preferred that the peel force of the adhesive face is max. about 4N/cm.

In one embodiment, the adhesive layer forms part of a wound dressing which also comprises an absorbent core for absorbing wound exudate. The absorbent core and/or the adhesive gel may contain active ingredients, such as ibuprofen, paracetamol, silver or other medically active ingredients adapted to kill pain or to improve the healing of a wound.

Initial viscosity is preferably 0.1 to 1000 Pa·s, more preferably 0.5 to 100 Pa.s, such as 0.5 to 50 Pa·s.

Gelation is between 0 and 60 min., more preferably between 0.5 and 30 min at 25 to 130°C, to allow good coating processing (i.e. within less than a few minutes) and separation of the two coated layers.

During manufacture, the adhesive material may reach a form-stable state without being fully reacted.

In the context of the present invention, the term "Form-stable" means that the material retains its shape under normal conditions, i.e. in the temperature range 25-130°C.

Full reaction or gelation may occur at a subsequent step of post-curing at which thermal energy may be provided to accelerate full reaction.

The invention according to the first aspect may also be described in the following manner:
Embodiment 1: A method for producing apertures in an adhesive, comprising the steps of:
   (1) Coating the adhesive onto two backing layers;
   (2) Separating the two backing layers thereby producing one patterned adhesive layer on each backing layer.
Embodiment 2: The method according to embodiment 1 wherein the two backing layers are held together in step (1) preferably by tension or by a thin adhesive layer.
Embodiment 3: The method according to any of the embodiment 1-2, wherein the first adhesive layer has perforations and the second adhesive layer comprising discrete adhesive particles of the same shape as the perforations of the first adhesive layer.
Embodiment 4: The method according to any of the embodiments 1-3, wherein the two adhesive layers comprising the same adhesive material.
Embodiment 5: The method according to any one of embodiments 1-4, wherein the adhesive edges around the holes of the perforated backing layer are formed while the adhesive is a liquid and flowable.
Embodiment 6: The method according to any one of embodiments 1-5, wherein the method further comprises curing the adhesive after step (2).
Embodiment 7: The method according to any one of embodiments 1-6, wherein at least one of the backing films may be perforated.
Embodiment 8: A method of making two adhesive layers with two different adhesive patterns simultaneously, wherein the first layer of adhesive is a connected layer, and the second adhesive layer comprises discrete dots or areas of adhesive.
Embodiment 9: The method according to embodiment 8, wherein the first layer of adhesive is a connected layer with perforations.
Embodiment 10: A wound dressing comprising both of the two adhesive layers manufactured according to any one of embodiments 1-9.

The invention according to the first aspect may comprise any combination of features and/or elements of the invention according to the second and/or third and/or fourth aspect.

In a SECOND aspect the present invention relates to a backing layer product comprising a backing layer carrying an adhesive layer, wherein the backing layer product is made according to the method of the first aspect of the invention.

The invention according to the second aspect may comprise any combination of features and/or elements of the invention according to the first and/or third and/or fourth aspect.

In a THIRD aspect, the present invention relates to a wound dressing comprising a first and second adhesive layer, the two adhesive layers being manufactured according to the method according to the first aspect of the invention.

The invention according to the third aspect may comprise any combination of features and/or elements of the invention according to the first and/or second and/or fourth aspect.

### DETAILED DESCRIPTION OF THE FIGURES

The invention will now be described in further detail with reference to the figures.

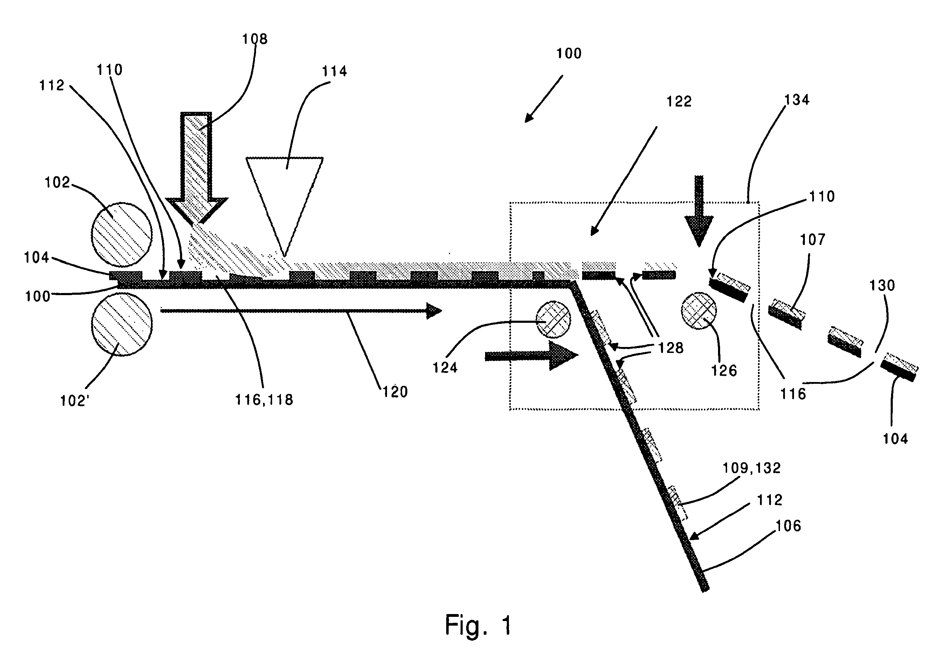

A continuous manufacturing method of the invention may have a set up as illustrated in Figure 1.

Fig. 1 discloses an apparatus 100 for carrying out the method according to the present invention. The apparatus comprises a set of rollers 102,102', which are arranged to press the first (upper) backing layer 104 and the second (lower) backing layer 106 together by pressing the layers towards each other. Downstream the rollers 102,102' a feeding unit 108 is provided. The feeding unit 108 is adapted to feed an adhesive in liquid form onto the upper surfaces 110,112 of the first and second backing layer 104,106 respectively. Downstream the feeding unit 108 a levelling unit 114 is arranged to even out and level the adhesive layer 107,109 such that it is evenly distributed across the upper surfaces 110,112.

As it will be appreciated from Fig. 1, the first (upper) backing layer 104 defines a plurality of indentations 116, allowing a part of the adhesive to flow on to predetermined areas 118 on the upper surface of the second (lower) backing layer. Upon further movement (indicated by arrow 120) of the two backing layers 104,106, the layers 104,106 reaches a separation point 122 at which the two adhesive layers 107,109 are moved away from each other such that the layers 107,109 are separated. This causes the first adhesive layer 107 (defined on the upper surface of the first backing layer) and the second adhesive layer 109 (defined on the upper surface of the second backing layer) to be separated.

At the separation point 122 a roller 124 is provided and about which the second (lower) backing layer 106 is moved/pulled in a direction away from the first (upper) backing layer 104. A further roller 126 is provided further downstream relative to the direction of movement of the first backing layer 104. The roller 126 is used to lead the first backing layer in a downwards direction. In one embodiment, the rim portion 128 of each of the perforations 130 defined in the first (upper) adhesive layer 107 and the rim portion of the adhesive zones 132 defined in the second adhesive layer 109, are defined when said rim portions have reached a form-stable state. In other embodiments, the two layers are separated prior to the rim portions 128 have reached their form stable state.

Two sheets of backing film 104,106 are pressed and held together by tension.

This could be between two rollers 102,102' before the addition of adhesive liquid 107,109. A further roller 124 may be provided below the backing layer at a point downstream the coating and upstream the point of separation 122. Downstream the separation point 122, the two layers 104,106 are directed downwards relative to the direction of the layers 104,106 upstream the separation point 122. The two layers 104,106 may be advanced at a slight tension in order to ensure that the two backing layers 104,106 are firmly held together along the length of the conveyor before separation point 122, i.e. upstream said point. The key is that the top coated surface is free or it is in contact with tension controllers, e.g. rollers above, only at areas which are outside the adhesive surface for use in making dressings, e.g. on the peripherals of the film rolls.

Alternatively the two layers may be held together by a thin adhesive layer or any other known means.

Liquid adhesive of initially low viscosity is added by means of the feeding unit 108, which may utilise any conventional methods such as by being poured out through a slit-shaped opening. Pumps and static mixers may be provided for feeding the liquid out through the slit-shaped opening.

In some embodiments, the surface of the applied layer is smoothened prior to being subjected to the moulding tool. Smooth coating at the right thickness may be achieved by any known means, e.g. die slot, doctors knife. Alternatively, or as a supplement, the press may be used to ensure the right spread and coating thickness.

Within the area depicted in the square 134, the backing layers 107,109 are separated. The separation of the two adhesive layers 107,109 should be fast (equivalent to the advancing speed of the two backing layers) so as not to allow flow or leave strings of adhesive between the adhesive layers 107,109, i.e. from the indentations 116 of the first adhesive layer 107 to the adhesive zone 132 of the second adhesive layer 109. After separation, the gelation, or increase in viscosity of the adhesive material to form-stable state should be so fast that flow into uncoated or perforated areas is avoided.

The energy source for providing curing energy may be any suitable source, e.g. microwaves, heat, UV-radiation, IR-radiation etc. and may be applied from any direction, e.g. top or bottom.

Post-curing of the form-stable adhesive material is also a possibility.

In one embodiment of the invention the first adhesive layer 107 defines perforations 130 and the second adhesive layer 109 comprises discrete adhesive zones 132 corresponding in shape to the perforations 130 of the first adhesive layer 107.

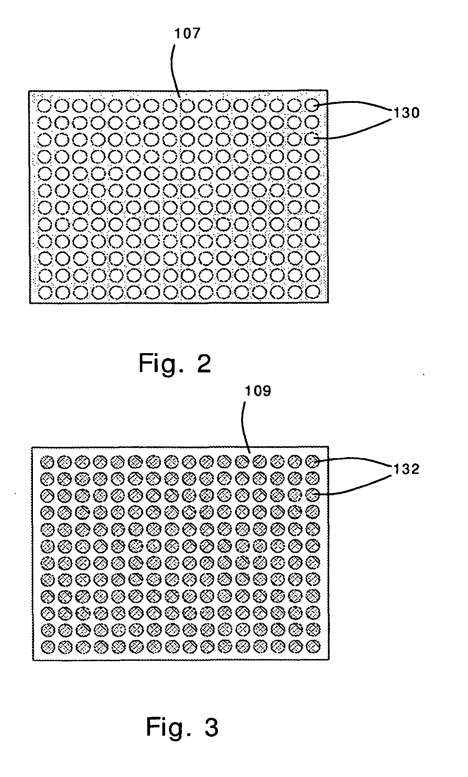

Figure 2 illustrates a top view of a top coated layer manufactured by the method illustrated in Figure 1.

Perforations/holes 130 which may be of any shape or size, may be distributed in any way desirable. The edges/rim portion 128 of the perforations/holes 130 may in some of the embodiments be characterised in that the adhesive edge/rim portion 128 is formed while the adhesive is a liquid and flowable.

In one embodiment the perforations/holes 130 are preferably 0.1 to 100 mm in diameter. The centres of any two neighbouring the perforations/holes may be spaced apart at a distance of 0.4 to 10 mm.

Figure 3 illustrates a top view of a bottom coated layer manufactured by the method illustrated in Figure 1.

The adhesive zones/dots 132 may be of any shape and size may be distributed in any way desirable. However, as the adhesive flows onto the second backing layer 106 through the indentations 116 of the first backing layer 104, the adhesive zones 132 will coincide with the perforations/holes 130 of the first (top/upper) layer 104. The rim portions/edges 128 of the adhesive zones/dots 132 may in some embodiments be formed while the adhesive is a liquid and flowable. Furthermore, the backing film 104 may be perforated at uncoated areas thereof.

The backing films 104,106 may be organic or synthetic, woven or non woven materials. The backing films 104,106 may be patterned or textured.

In one embodiment, an adhesive sheet 107,109 comprises an adhesive defining a predetermined pattern which is made according to the method of the present invention.

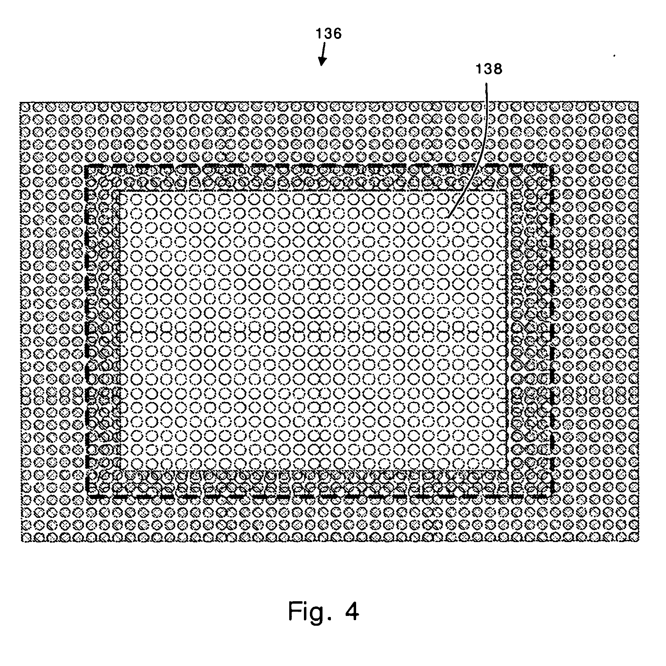

Fig. 4 discloses a wound dressing 136 comprising a backing layer 104,106 on which an absorbent core 138 is provided and on which an adhesive layer 107,109 may be provided.

The dressings 136 made from any single type of pattern or perforation have advantages and drawbacks. The use of different patterns at different parts of the dressing construction can reduce some of these drawbacks.

A preferred construction of a dressing 136 utilising the advantages to the two types of adhesive sheets 107,109 formed by the method according to the invention, is illustrated in Figure 4.

In one embodiment of the invention a wound dressing 136 comprising both of the two adhesive layers 107,109 manufactured according to method of the invention.

The dressing 136 may define any shape, e.g. round or rectangular.

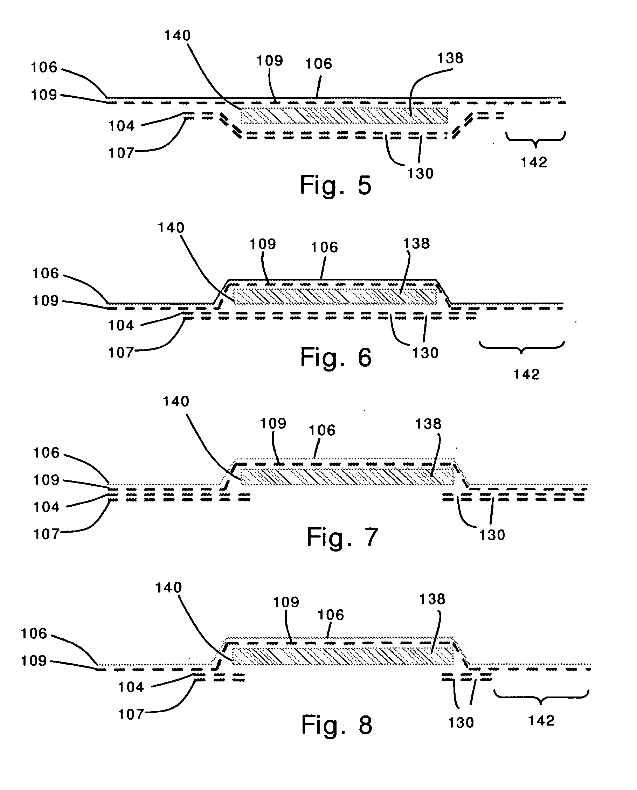

The dressing of Fig. 4 comprises a piece of the second backing layer 106 (i.e. with discontinuous dots/adhesive zones 132), a piece of the first backing layer 104 (i.e. defining indentations 116 and an adhesive layer 107) and an absorbent core 138 provided between the first and second backing layers 104,106 as is illustrated in Figs. 5-8. In use the adhesive of the first adhesive layer 107 (i.e. which is adhered to the first backing layer 104) faces the wound.

The dressing 136 is characterised in that the first backing layer 104 has a diameter which is equal to or slightly larger than that of the absorbent core 138. The first adhesive layer 107 and the first backing layer 104, defines a plurality of indentations 116 and perforations 130, respectively. As the adhesive does not define passages/channels between the perforations 130, the adhesive layer 107 eliminates leakage in the longitudinal direction of the layer 107 while allowing exudated to flow in a direction transverse to the longitudinal direction of the adhesive, i.e. through the indentations 116 and the perforations 130 and into the absorbent core 138.

The absorbent core 138 is attached to the backing layer 106 by any known method, preferably by adhesive or welding. In one embodiment, the adhesive 109 provided on the backing layer 106 is used to adhere the absorbent core 138 to the backing layer 106. The absorbent core 138 may be fastened (by adhesive and/or welding) to the backing layer 106, in selected areas of the core 138. Alternatively, the entire surface of the absorbent core 138 is fastened (adhesive and/or welding) to the backing layer 106. The edges 140 of the absorbent core 138 may be adhered or welded to the second backing layer 106.

The first backing layer 104 is attached to the wound facing surface of second backing layer 106 and to the absorbent core 108 as is illustrated in Fig. 5-8. This attachment may be achieved by any known method, preferably by adhesive or welding. In one embodiment, the first backing layer 104 is orientated such that the first adhesive layer 107 faces away from the wound during use and is used to adhere the first backing layer 104 to the wound facing surfaces of the absorbent core 138 and/or the second backing layer 106. The adhesive or welding which attaches the first backing layer 104 to the second adhesive layer 109 may be defined at least at the outer edge/rim of the first backing layer 104, and may in some embodiments define a seal between the first and second backing layer 104,106. It will be appreciated that adhesives which during use do not face the skin or wound need not be skin friendly. Moreover, the latter kind of adhesives may be stronger than adhesives for attachment to the skin, especially in cases where such adhesives are used to adhere two elements of the dressing together.

Figure 5 illustrates a sectional view taken along a line in the middle from one edge to the opposite edge of the dressing illustrated in Figure 4.

Figure 6 illustrates a sectional view of another preferred construction where the outer layer 106 defines an indentation for receipt of the absorbent c that the wound facing surface is flat.

Figure 7 illustrates a sectional view of another preferred construction.

The layer 104 may slightly covering slightly the edge of the core 138 for added support, leaving the central part of the core uncovered.

As may be seen in Fig. 7, the first backing layer 104 may also be ring-shaped with a outer diameter corresponding to that of the second backing layer 107 whereby the two layers overlap in the outer rim portion of both the backing layers 104,107. This may increase the stiffness of the wound dressing member 136 which is useful during application of the dressing to the wound.

In Figs. 5, 6 and 8 the wound dressing 136 defines a ring-shaped one layer adhesive region 142, indicated by bracket 142. In the area of the ring-shaped one layer adhesive region 142, only one of the first and second backing layer 104,106 is provided. Accordingly the first and second backing layers 104,106 do not overlap in this region. Instead, the wound dressing 136 is only defined by the first backing layer 106 and the first adhesive layer 109. An advantage of this configuration is that it provides better permeability and peel friendliness of in the area of the ring-shaped one layer adhesive region 142.

In one embodiment, a ring-shaped first backing layer 104is provided in the transition between the absorbent core 138 and the second backing layer 106, whereby a seal is provided which prevents lateral leakage from the absorbent core 138, see Fig. 8. Such a ring-shaped first backing layer 104 may be welded onto the wound facing surfaces of the absorbent core 138 and the second backing layer 106, such that the first adhesive layer 107 of the first adhesive layer 104 faces the wound during use. Alternatively, an adhesive is provided on both surfaces of the first adhesive layer 104 - one which is used to adhere the first adhesive layer 104 to the skin/wound and a second which attaches the first backing layer to the absorbent core 138 and the second backing layer 106.

It will be appreciated that any surface coated with an adhesive, which during normal use of the wound dressing will not contact the skin of a user may be any type of adhesive (or welding), thus having lower requirements to skin friendliness.

The two adhesive layers produced by the invention may also be used separately.

For example, another construction may comprise the second backing layer 106 and a centrally positioned absorbent layer 138.

In a embodiment, of the invention the liquid permeable layer is a ring layer around the borders of the upper layer covering at least partially the border.

In another embodiment, the liquid permeable layer is a ring layer around the borders of the upper layer.

### EXAMPLES

The invention will now be described by way of the following examples:

### Materials

The following materials were used in the examples
- Siliconised PET liner: PET 1876 50µm with 1778 silicone supplied by Huhtamaki
- Primer: CF1-135, supplied by Nusil
- PU film: Bioflex 130, 25 µm, supplied by Scapa
- Silicone gel adhesive: Silbione RTgel 4512, supplied by Rhodia
- Acrylat: free standing RX1240ULT, supplied by Scapa.
- Mask: PP2500 100m, supplied by 3M .
- Construction adhesive: Re Mount, supplied by3M
- PE liner: PE 16000, supplied by Huhtamaki
- Siliconised PE: PE16000-803, supplied by Huhtamaki

### Laminate construction procedure:

Option one: Discrete pattern coated silicone or any curable adhesive such as polyurethane, polyacrylate and the like, provided on a release liner** was transfer coated onto a PU film supported by a backing paper, the release liner was removed afterwards, and the Moisture Vapour Transmission Rate (MVTR) was measured according to DS/EN 13726-2 standard.

Option two: Dot coated silicone adhesive was provided on a release liner, the latter was transferred onto a permanent substrate (i.e. a substrate for use in a final product and not a release liner) such us PU film or a polyurethane foam, silicone foam or any foamable material. After transfer coating, the MVTR was measured according to the standard mentioned above.

Option three: Dots or discrete pattern coated silicone provided on a permanent substrate was laminated onto another substrate by means of heat or adhesion. After lamination, the MVTR of the hole construction (i.e. the first adhesive layer defining a plurality of perforations) was measured according to the standard mentioned above.

** By a release liner it is understood; a liner from which the adhesive is easily removed, the adhesive being coated onto release linier during manufacture. The removal is carried out by means transfer coating (i.e. an adhesive provided between two release liners), or another means known in the art.

The start permeability of the substrates used in the construction of the following examples, wherein

### Permeability of the PU film

| Sample | Perm. g/m², 24 hours |
|---|---|
| | 1126,6 |
| | 1260 |
| Bioflex 130 primer | 1297,1 |
| | 1126,8 |
| | 1232,1 |
| Average | 1229 |
| STDEV | 73,16 |

Permeability of a PU film coated* with 12 microns of Acrylate adhesive in predetermined pattern, defining a 25 % adhesive free area on the release liner

| Sample | Time (hours) | Perm. g/m², 24 hours |
|---|---|---|
| | 24 | 788,62 |
| Bioflex 130 acrylate coated in pattern | 24 | 882 |
| | 24 | 907,97 |
| | 24 | 788,76 |
| | 24 | 862,47 |
| Average | | 860,3 |
| STDEV | | 51,21 |

* Coating procedure: Free standing Acrylate adhesive was gravure coated in a predetermined pattern onto a siliconised release liner, the coat weight was 12 g/m2 and the predetermined pattern defined a 25% adhesive free area on the release liner. By transfer coating technique the free standing Acrylate adhesive was transferred onto a PU film, the siliconised paper was kept on until further use.

### Example 1 (Reference example)

Silicone gel adhesive composed of part A (divinyl polydimethysiloxane (PDMS) polymer and the Pt based catalyst) and part B(rosslinker Si-H terminated PDMS), was mixed together in a 1:1-ratio and coated onto a thermoplastic polyurethane film (PU). The PU film was a 25 microns PU film The thickness of the coating was 200 µm, after curing the MVTR was measured according to DS/EN 13726-2. The result is provided in the below table.

| Sample | Perm. g/m², 24 hours |
|---|---|
| PU film coated with a full coverage silicone gel | 286 |
| | 293 |
| | 317 |
| Average | 299 |

### Example 2

Two PU film carriers of same thickness (25 µm), a first comprising circular perforations of 3 mm in diameter. The perforations constituting 30% of the total area of the film. A second PU film carrier without perforations, the two carriers had a paper backing of 100 microns in thickness. The two carriers were temporarily laminated together by adhesion. The laminate comprising the two carriers were positioned on a coating board such that the carrier defining perforations were facing away from the coating board and the carrier which did not define perforations faced the coating board. A 200 µm silicone gel adhesive was coated onto the laminate and cured. After curing, the laminated carriers were separated so as to obtain a PU carrier (the lower of the two) where the silicone was provided as dot-shaped adhesive zones of 3 mm in diameter and 125 microns in thickness and a PU film (the upper of the two) where the silicone gel was provided as a layer comprising perforations of 3 mm in diameter.

The backing paper of the upper adhesive (defining the sheet of adhesive with the perforations) was removed and subsequently laminated by adhesion onto PU film priory pattern coated with a 12 µm Acrylate adhesive . The MVTR was measured afterwards, the results are presented in the table below.

Discrete inter-connected pattern coated substrate in a laminate construction

| Sample | Time (hours) | Perm. g/m² 24 hours |
|---|---|---|
| Discrete inter-connected pattern coated PU film with silicone gel | 24 | 497,5 |
| | 24 | 437,9 |
| | 24 | 467,5 |
| | 24 | 461,2 |
| | 24 | 512,1 |
| | 24 | 431,6 |
| | 24 | 539,8 |
| Average | | 475,02 |
| STDEV | | 39,82 |

The PU film with dot-shaped adhesive zones were laminated by means of heat onto a Polyurethane film and the MVTR was measured, the results are presented in the table below.

MVTR with dot-shaped adhesive zones coated onto a substrate to form a laminate construction

| Sample | Time (hours) | Perm. g/m² 24 hours |
|---|---|---|
| Coated PU film where the silicone gel lies as a discrete dots | 24 | 488,3 |
| | 24 | 527,7 |
| | 24 | 524,9 |
| | 24 | 581,3 |
| | 24 | 523,5 |
| | 24 | 573,9 |
| | 24 | 621,8 |
| Average | | 558,85 |
| STDEV | | 45,34 |

It will be appreciated that the thickness of the discrete dot-shaped adhesive zones is determined by the thickness of the liner which during manufacture is provided above the liner on which the adhesive zones are formed.

It will be appreciated that the scope of the invention is not limited to the techniques and materials used in the examples.

Backing layers/films may include vapour permeable or impermeable films, textiles, non wovens, nets which may include an extra support carrier on the non coating side.

In order to manufacture an adhesive which is transferable or an adhesive layer without a fixed backing film, the backing film could be vapour permeable or impermeable films, such as siliconised release liners, PE, PP, PET, which are easily removable from the adhesive layer.

After processing, the adhesive layer may be transferred to an alternative film by any means known to the skilled person or be used as an un-re-enforced adhesive layer. The two backing films may be two release liners as is described in the aforementioned. The adhesive may also further be re-enforced with a net by coating the adhesive onto the net placed on the backing film.

In order to ease the separation of the two layers during manufacture, especially with due regard to protecting the integrity of the dot patterns of the lower layer when the upper layer is being lifted, the lower backing layer may be pre-primed with a primer, e.g. CF1-135 from Nusil, to improve the anchoring of the dots.

In order to remove air bubbles in the adhesive due to mixing, the uncured liquid adhesive mixture may be subjected to vacuum so as to remove the air bubbles before application to the laminated backing films.

In order to hold the laminate in place during the coating process, it will be appreciated that any means to hold the substrate may be used, e.g. mechanical grips, mechanical or chemical means of adhesion, and vacuum suction.

## Claims

1. A method for manufacturing a first and a second backing layer each of which carrying on an upper surface an adhesive layer defining a predetermined pattern, the first backing layer defining one or more indentations through each of which an adhesive coated onto the upper surface of the first backing layer may flow onto a predetermined area of the upper surface of the second layer when the first backing layer is provided on the upper surface of the second backing layer, the method comprising the steps of:
- providing the first backing layer on the upper surface of the second backing layer;
- coating the adhesive onto the upper surface of the first backing layer and the predetermined areas of the second backing layers;
- separating the first and second backing layers so as to define the predetermined patterns on the upper surface of each of the first and second backing layer.

2. The method according to claim 1, wherein the step of coating the adhesive comprises the step of retaining the first backing layer on the upper surface of the second backing layer.

3. The method according to any of the claims 1-2, wherein the step of separating causes:
- a first adhesive layer to be defined on the upper surface of the first backing layer, the first adhesive layer defining a perforation in the area of each of the indentations in the first backing layer; and
- a second adhesive layer to be defined on the upper surface of the second backing layer, the second adhesive layer defining discrete adhesive zones in each of the predetermined areas of the second backing layer.

4. The method according to any of the claims 1-3, wherein the first and second adhesive layer comprises the same adhesive material.

5. The method according to any one of the preceding claims, wherein each of the perforations in the first adhesive layer defines a rim portion, and wherein said rim portion is defined while the rim portion is in liquid form.

6. The method according to any one of the preceding claims, wherein the method subsequent to the step of separating comprises the step of curing the first and second adhesive layer.

7. The method according to any of claims 1-4, wherein each of the perforations in the first adhesive layer defines a rim portion, and wherein said rim portion is defined when the rim portion is in a form-stable form.

8. The method according to any of claims 1-4 or 7, wherein the method prior to the step of separating comprises the step of curing the first adhesive layer at least in the area of the rim portion.

9. The method according to any of the preceding claims, wherein the method prior to the step of coating comprises the step of priming at least a part of the upper surface of the first and/or second backing layer with a primer adapted to enhance the binding between the adhesive and the respective surface(s).

10. A wound dressing comprising a first and second adhesive layer, the two adhesive layers being manufactured according to any one of claims 1-9.

## Patentansprüche

1. Verfahren zur Herstellung einer ersten und einer zweiten Trägerschicht, die jeweils auf einer Oberseite eine ein vorbestimmtes Muster definierende Klebeschicht tragen, wobei die erste Trägerschicht ein oder mehr Einbuchtungen definiert, durch die jeweils ein auf der Oberseite der ersten Trägerschicht aufgetragener Klebstoff auf einen vorbestimmten Bereich der Oberseite der zweiten Schicht fließen kann, wenn die erste Trägerschicht auf der Oberseite der zweiten Trägerschicht bereitgestellt wird, wobei das Verfahren die folgenden Schritte umfasst:
- Bereitstellung der ersten Trägerschicht auf der Oberseite der zweiten Trägerschicht;
- Aufbringen des Klebstoffs auf der Oberseite der ersten Trägerschicht und den vorbestimmten Bereichen der zweiten Trägerschicht;
- Trennen der ersten und zweiten Trägerschichten, um die vorbestimmten Muster auf der Oberseite der ersten und der zweiten Trägerschicht zu definieren.

2. Verfahren nach Anspruch 1, worin der Schritt des Aufbringens einer Klebstoffschicht den Schritt des Haltens der ersten Trägerschicht auf der Oberseite der zweiten Trägerschicht umfasst.

3. Verfahren nach einem der Ansprüche 1-2, worin der Trennschicht folgendes verursacht:
- eine erste auf der Oberseite der ersten Trägerschicht zu definierende Klebeschicht, wobei die erste Klebeschicht eine Perforation im Bereich jeder Einbuchtung in der ersten Trägerschicht definiert; und
- eine zweite auf der Oberseite der zweiten Trägerschicht zu definierende Klebeschicht, wobei die zweite Klebeschicht diskrete Klebezonen in jedem der vorbestimmten Bereiche der zweiten Trägerschicht definiert.

4. Verfahren nach einem der Ansprüche 1-3, worin die ersten und zweiten Klebeschichten dasselbe Klebematerial umfassen.

5. Verfahren nach einem der vorhergehenden Ansprüche, worin jede der Perforationen in der ersten Klebeschicht einen Randabschnitt definiert und worin der Randabschnitt definiert ist, während sich der Randabschnitt in flüssiger Form befindet.

6. Verfahren nach einem der vorhergehenden Ansprüche, worin das Verfahren nach dem Trennschritt den Schritt des Aushärtens der ersten und zweiten Klebeschicht umfasst.

7. Verfahren nach einem der Ansprüche 1-4, worin jede der Perforationen in der ersten Klebeschicht einen Randabschnitt definiert und worin der Randabschnitt definiert ist, während sich der Randabschnitt in einer formstabilen Form befindet.

8. Verfahren nach einem der Ansprüche 1-4 oder 7, worin das Verfahren nach dem Trennschritt den Schritt des Aushärtens der ersten Klebeschicht zumindest im Bereich des Randabschnitts umfasst.

9. Verfahren nach einem der vorhergehenden Ansprüche, worin das Verfahren vor dem Beschichtungsschritt den Schritt des Grundierens zumindest eines Teils der Oberseite der ersten und/oder zweiten Trägerschicht mit einem Grundiermittel, das die Bindung zwischen dem Klebstoff und der bzw. den jeweiligen Fläche(n) verbessert, umfasst.

10. Wundverband, der eine erste und eine zweite Klebeschicht umfasst, wobei die beiden Klebeschichten nach einem der Ansprüche 1-9 hergestellt wurden.

## Revendications

1. Procédé de fabrication d'une première et d'une deuxième couches de dossier portant chacune sur une surface supérieure une couche d'adhésif qui définit un motif prédéterminé, la première couche de dossier définissant une ou plusieurs indentation(s) à travers chacune desquelles un adhésif déposé sur la surface supérieure de la première couche de dossier peut s'écouler sur une région prédéterminée de la surface supérieure de la deuxième couche de dossier lorsque la première couche de dossier est disposée sur la surface supérieure de la deuxième couche de dossier, le procédé comprenant les étapes suivantes:
- disposer la première couche de dossier sur la surface supérieure de la deuxième couche de dossier;
- appliquer l'adhésif sur la surface supérieure de la première couche de dossier et sur les régions prédéterminées de la deuxième couche de dossier; et
- séparer les première et deuxième couches de dossier de manière à définir les motifs prédéterminés sur la surface supérieure de chacune des première et deuxième couches de dossier.

2. Procédé selon la revendication 1, dans lequel l'étape d'application de l'adhésif comprend l'étape de retenue de la première couche de dossier sur la surface supérieure de la deuxième couche de dossier.

3. Procédé selon l'une quelconque des revendications 1 à 2, dans lequel l'étape de séparation entraîne:
- une première couche d'adhésif à être définie sur la surface supérieure de la première couche de dossier, la première couche d'adhésif définissant une perforation dans la région de chacune des indentations dans la première couche de dossier; et
- une deuxième couche d'adhésif à être définie sur la surface supérieure de la deuxième couche de dossier, la deuxième couche d'adhésif définissant des zones adhésives discrètes dans chacune des régions prédéterminées de la deuxième couche de dossier.

4. Procédé selon l'une quelconque des revendications 1 à 3, dans lequel les première et deuxième couches d'adhésif comprennent la même matière adhésive.

5. Procédé selon l'une quelconque des revendications précédentes, dans lequel chacune des perforations dans la première couche d'adhésif définit une partie de rebord, et dans lequel ladite partie de rebord est définie alors que la partie de rebord se trouve sous une forme liquide.

6. Procédé selon l'une quelconque des revendications précédentes, dans lequel le procédé après l'étape de séparation comprend l'étape de durcissement des première et deuxième couches d'adhésif.

7. Procédé selon l'une quelconque des revendications 1 à 4, dans lequel chacune des perforations dans la première couche d'adhésif définit une partie de rebord, et dans lequel ladite partie de rebord est définie lorsque la partie de rebord se trouve sous une forme stable.

8. Procédé selon l'une quelconque des revendications 1 à 4 ou 7, dans lequel le procédé, avant l'étape de séparation, comprend l'étape de durcissement de la première couche d'adhésif au moins dans la région de la partie de rebord.

9. Procédé selon l'une quelconque des revendications précédentes, dans lequel le procédé, avant l'étape d'application, comprend l'étape d'application d'une couche de fond sur au moins une partie de la surface supérieure de la première et/ou de la deuxième couche(s) de dossier en utilisant un primaire qui est adapté pour améliorer la liaison entre l'adhésif et la (les) surface(s) respective(s).

10. Crêpe-pansement comprenant une première et une deuxième couches adhésives, les deux couches adhésives étant fabriquées selon l'une quelconque des revendications 1 à 9.
